(19) Europäisches Patentamt
European Patent Office
Office européen des brevets

(11) Publication number: 0 328 121 A1

(12) **EUROPEAN PATENT APPLICATION**

(21) Application number: 89102266.7

(22) Date of filing: 09.02.89

(51) Int. Cl.⁴: C07D 487/14 , A61K 31/40 , //(C07D487/14,209:00,209:00, 203:00)

(30) Priority: 11.02.88 US 154891

(43) Date of publication of application: 16.08.89 Bulletin 89/33

(84) Designated Contracting States: AT BE CH DE ES FR GB GR IT LI LU NL SE

(71) Applicant: UNIVERSITY OF HOUSTON-UNIVERSITY PARK
4800 Calhoun Road
Houston Texas 77004(US)

(72) Inventor: Kohn, Harold L.
3735 Latma Drive
Houston Texas 77025(US)

(74) Representative: Patentanwälte Grünecker, Kinkeldey, Stockmair & Partner
Maximilianstrasse 58
D-8000 München 22(DE)

# (54) Substituted hydrazine mitomycin analogs.

(57) 7-substituted hydrazine mitomycin analogs are provided having anti-tumor activity in experimental animal tumors. The compounds are made by treating mitomycin A with a substituted acid hydrazide or alkyl carbazate. Acyl substituted hydrazine mitomcyin analogs are disclosed.

EP 0 328 121 A1

## SUBSTITUTED HYDRAZINE MITOMYCIN ANALOGS

The present invention relates to mitomycin analogs having a substituted hydrazine at position 7. These compounds are active anti-tumor substances having in vivo inhibitory activity against experimental animal tumors.

Mitomycin C is an antibiotic which is produced by fermentation and is presently on sale under Food and Drug Administration approval in the therapy of disseminated adenocarcinoma of the stomach or pancreas. (Mutamycin® Bristol Laboratories, Syracuse, New York 13201, Physicians' Desk Reference 42d Edition 1988, pages 776-777.) Mitomycin C and its production by fermentation is the subject of U.S. Patent No. 3,660,578 patented May 2, 1972.

The structures of mitomycins A, B, C and of porfiromycin were first published by J. S. Webb et al. of Lederle Laboratories Division American Cyanamid Company, J. Amer. Chem. Soc. 84:3185-3187 (1962). One of the chemical transformations used in this structure study to relate mitomycin A and mitomycin C was the conversion of the former, 7,9α-dimethoxymitosane, by reaction with ammonia to the latter, 7-amino-9α-methoxymitosane. Displacement of the 7-methoxy group of mitomycin A has proven to be a reaction of considerable interest in the preparation of anti-tumor active derivatives of mitomcyin. The prior art, however, has not provided any example of a 7-substituted hydrazine mitomycin analog of the sort provided by the present invention.

Extensive studies on the mechanism of action of mitomycin C have shown that reduction of the quinone moiety activates the drug and facilitates cross-linking of the drug to cellular DNA, the genetic material of the cell. Consistent with this hypothesis, mitomycin C functions best within poorly vascularized, hypoxic regions of tumors or infected tissue. These regions contain sufficient cellular reductases to initiate activation of the drug and promote binding of the drug to DNA.

Since curative therapy requires that all tumor cells or infectious bacteria be destroyed, a need exists for chemotherapeutic agents which function under a variety of cellular conditions. In the case of mitomycin, there exists a need for analogs which would function in tumor or tissue regions which have reduced levels of cellular reductases or are less hypoxic. The present invention provides such mitomycin analogs which may possess greater bioactivity in cellular regions which are less hypoxic.

The present invention provides mitomycin analogs having a substituted hydrazine substituent at the 7-position of the mitomycin ring structure. These compounds can be represented by the following formula:

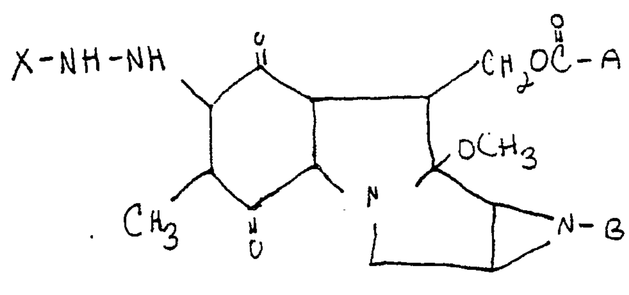

or its corresponding tautomeric form,
wherein:
X is selected from the group consisting of:

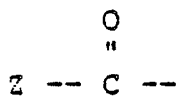 ; 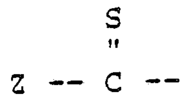 ; 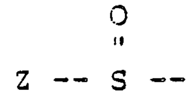 ;

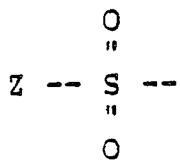 ; and 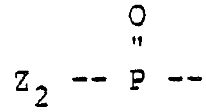 ;

wherein Z is R or RY;
wherein R is hydrogen, lower alkyl, lower alkenyl, lower alkynyl, aryl lower alkyl, aryl, aryl lower alkenyl, aryl lower alkynyl, heterocyclic, heterocyclic lower alkyl, polynuclear aromatic or polynuclear aromatic lower alkyl, each unsubstituted or substituted with one or more halogen or a heteroatom such as oxygen, nitrogen, sulfur or phosphorous substituted with hydrogen, lower alkyl or aryl; carboyhydrate; amino acid; nucleic acid; oligo peptide or protein; and
Y is NH, O, or S.

The present invention also contemplates the use of the substituted hydrazine as a linker of other drug candidates to the mitomcyin anti-tumor moiety. For example, these drug candidates may be other antineoplastic agents, toxins, enzymes, or organic chemical drugs. This binary system would offer greater overall drug selectivity and sensitivity thereby reducing the adverse toxic effects of either mitomycin or the drug given along. Examples of such a binary system would include X substituted with a phosphoramidase substrate; polypeptide plasmin substrates, such as Boc-Val-Leu-Lys-; nitrosoureas; steroids such as prednisolone; or DNA-intercalating agents, such as adriamycin, actinomycin D or ellipticine.

In the above mitomycin formula, the various substituents for A and B have been described previously in the patent and scientific literature. See, e.g., United States Patent Nos. 4,691,023; 4,642,352; 4,590,074; 4,567,256; 4,460,599; or Fishbein and Kohn, J. Med. Chem. 30:1767 (1987). In general, by way of description and without intent to limit the substituents, A and B can be defined as: A is $R_1$ or $YR_1$, wherein $R_1$ includes:
hydrogen, lower alkyl, lower alkenyl, lower alkynyl, aryl, each unsubstituted or substituted with one or more of halogen or a heteroatom such as oxygen, nitrogen, sulfur or phosphorous substituted with hydrogen, lower alkyl or aryl;
and Y is NH, O, or S; and

B is $R_1$ ; 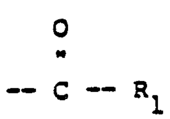 ; 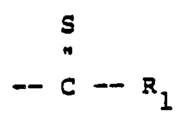 ;

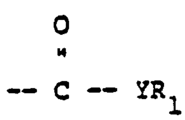 ; 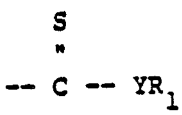 ; 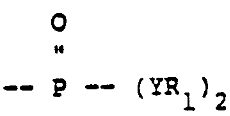 ;

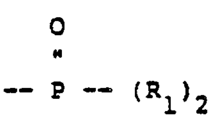 ; 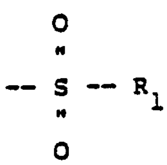 ; 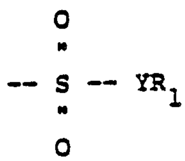 ; or 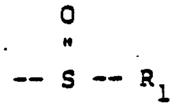 .

Further, as to the stereochemical configuration, the mitomycin derivatives of this invention retain the stereochemical configuration the same as that of mitomycin C.

These substituted hydrazine-7-mitomycin compounds are inhibitors of experimental tumors in animals. They are comparable to mitomycin C with respect to the types of tumors which they inhibit, and possess similar activity achieved with mitomycin C. For anti-tumor purposes, they are administered to a mammal bearing a tumor in substantially non-toxic anti-tumor effective dose.

They are administered primarily by injection in much the same way as mitomycin C. They are readily distributed as dry pharmaceutical compositions containing diluents, buffers, stabilizers, solubilizers, and ingredients contributing to pharmaceutical elegance. These compositions may be constituted with an injectable liquid extemporaneously just prior to use. Suitable injection liquids include water, isotonic saline, etc.

The compounds of this invention are prepared by reacting an acid hydrazine or alkyl carbazate with a mitomycin derivative wherein the 7-position is a group readily displaceable by reaction with the acid hydrazide or alkyl carbazate. Specific examples of this invention provided in this disclosure have X substituted with acetyl; benzoyl; 3-pyridine carboxy; 2-thiophenyl carboxy; carbethoxy; carbo*tert*-butoxy; carbobenzoxy; or carbo-9-fluoromethyloxy.

By way of theory, which is not to be construed as a limitation of the invention, the incorporation of the substituted hydrazine reductant on mitomycin is thought to increase the anti-tumor bioactivity. It is through that the hydrazine reductant on mitomycin may decrease the need for cellular reductases normally required for the activation of the drug and permit the chemotherapeutic agent to function in regions of tumor growth which are less hypoxic.

## DETAILED DESCRIPTION OF THE INVENTION

The synthetic approach adopted for the preparation of the compounds of this invention was patterned after the procedure utilized by Remers and coworkers, J. Med. Chem. 26:1453-1457 (1983) for the synthesis of carbon-7 amino-substituted mitomycins. Mitomycin C was first converted to mitomycin A and then treated with two equivalents of the acid hydrazine or alkyl carbazate in methanol. Eight substituted mitomycins (I-VIII) were produced in this fashion. Each reaction proceeded in good to excellent yield and gave only a single product (TLC analysis).

Compounds referred to in the text and claims are based on the conventional numbering system used for mitomycins:

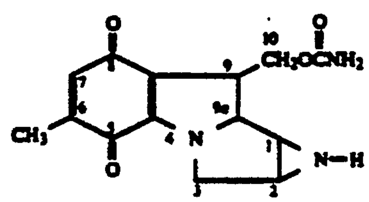

The names which appear in the examples are based on the current chemical abstract nomenclature system using the following ring structure:

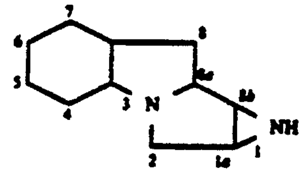

The hydrazino-mitomycin derivatives were all characterized in the solution state as the orthoazaquinone tautomer on the basis of several key spectral observations. First, consistent patterns were noted throughout the ultraviolet-visible, and $^1H$ and $^{13}C$ NMR spectra for these eight compounds, suggesting that these adducts contained a common structure. Second, compounds I-VIII all displayed a pronounced absorption in the visible spectrum between 376-395 nm. A comparable band of near equal intensity appears in mitomycin C at 360 nm $^{-1}$. Third, the carbon-6 methyl protons in I-VIII consistently appeared in the $^1H$ NMR spectra at ~$\delta$ 2.1. This value was downfield from the value (~$\delta$ 1.8) typically observed for mitomycin C adducts. Fourth, the $^1H$ NMR spectrum of II in $CD_3CN$ revealed a peak at $\delta$ 17.03 integrating for one proton. Signals in this downfield region are normally associated with acidic hydrogens, (i.e., phenolic, carboxylic acid protons). Fifth, the pattern of the signals in the proton-decoupled $^{13}C$ NMR spectra for the quinone carbons in I-VIII were atypical for mitomycin C adducts. In particular, only one signal was detected in the range normally

observed for quinone carbonyl carbon atoms (i.e., 175-178 ppm), while a new resonance appeared between 137.8-143.3 ppm. Confirmation of the proposed assignment was secured from the single-crystal X-ray structure determination of the tert.-butylcarbazate derivative VI. The crystal structure of VI clearly showed that the compound existed in the ortho-azaquinone form with an intramolecular hydrogen bond occuring between the hydrogen on the hydrazine beta nitrogen atom and the carbon-8 quinone oxygen atom.

Melting points were determined with a Thomas Hoover Unimelt apparatus and are uncorrected. Infrared spectra (IR) were run on a Perkin-Elmer 283 spectrophotometer and calibrated against the 1601 $cm^{-1}$ band of polystyrene. Absorption values are expressed in wave numbers ($cm^{-1}$). Ultraviolet spectra were recorded on Hitachi-100 spectrophotometer. Proton ($^1H$ NMR) and carbon ($^{13}C$ NMR) nuclear magnetic resonance spectra were taken on either a Nicolet NT-3000 or a General Electric QE-300 NMR instrument. Chemical shifts are in parts per million ($\delta$ values) relative to $Me_4Si$, and coupling constants (J values) are in hertz and are reported using the following abbreviations: s, singlet; d, doublet; t, triplet; q, quartet; m, complex multiplet; br, broad. Low resolution mass spectral data were obtained on a Bell and Howell 21-491 mass spectrometer at the University of Texas-Austin by Dr. John Chinn. $CH_4$ was used as the ionizing gas for all spectra obtained under negative CI conditions. Elemental analysis were performed by Spang Mircoanalysis Laboratory, Eagle Harbor, Michigan. Ethyl carbazate, tert.-butyl carbazate, acethydrazide, benzoic acid hydrazide, 2-thiophencarboxylic acid hydrazide and nicotinic acid hydrazide were obtained from Aldrich Chemical Company, Milwaukee, Wisconsin. Benzylcarbazate were obtained from Lancaster Synthesis Ltd., Windham, New Hampshire.

Preparation of Mitomycin C Analogs (General Procedure)

A methanol solution (50mL) of mitomycin A (100 mg, 0.286 mmol) and 0.573 mmol of alkyl carbazate or acid hydrazide was stirred at room temperature until thin layer chromatographic analysis indicated that there was no further change in the reaction. The solvent was removed under vacuum at room temperature and the crude product triturated with a 90:10 mixture of $CHCl_3$-MeOH (10 mL). The orange to dark brown pure crystalline material that remained after trituration was filtered. A second crop of the crystalline material was obtained by removal of solvent from the filtrate and trituration of the residue with chloroform. The remaining chloroform layer was then concentrated and purified by medium pressure chromatography on a silica gel column (2 cm X 16 cm) using the indicated solvent system for elution. In those cases where trituration of the residue did not yield pure products, the final product was purified by medium pressure chromatography.

Using this procedure, the following compounds were synthesized:

## EXAMPLE I

Acetic acid, [1aS-(1a$\alpha$, 8$\beta$, 8a$\alpha$, 8b$\alpha$)]-[8-[[(aminocarbonyl)oxy]methyl]-1,1a,2,8,8a,8b-hexahydro-4-hydroxy-8a-methoxy-5-methyl-7-oxoazirino [2 ,3 ;3,4]pyrrolo[1,2-a]indol-6(7H)-ylidene]hydrazide

Making use of acethydrazide (42.4 mg, 0.573 mmol) gave 140 g of an orange brown solid which on purification by trituration yielded 67 mg (59%) of an orange crystalline product: mp >200 °C; $R_f$ 0.30 (20% methanol-chloroform); IR (KBr) 1700, 1620, 1540, 1470, 1400, 1375, 1350, 1320, 1230, 1190, 1165 $cm^{-1}$; UV (MeOH) $\lambda$ max 379 nm ($\epsilon$ 23800); $^1H$ NMR ($CD_3OD$) $\delta$ 2.05 (s, 3H), 2.30 (s,3H), 2.86 (br s, 1H), 3.00 (br s, 1H), 3.20 (s, 3H), 3.58 (br dd, J=4.0, 11.2 Hz, 2H), 4.31 (br dd, J=10.4, 11.2 Hz, 2H), 4.68 (dd, J-4.0, 10.4, Hz, 1H); $^{13}C$ NMR (pyridine-$d_5$) 10.90, 19.88, 32.46, 37.35, 43.80 49.51, 50.73, 62.18, 106.82, 110.60, 121.00, 137.82, 142.57, 158.17, 159.81, 173.77, 174.86 ppm; mass spectrum, m/e (relative intensity) (negative CI) 407 (M+16, 18), 392 (M+1, 21), 391 (M, 100), 348 (7), 300 (7), 288 (10), 286 (15).

| | | | | | | |
|---|---|---|---|---|---|---|
| Anal. Calcd for $C_{17}H_{21}N_5O_6$: | C, | 52.17; | H, | 5.40; | N, | 17.89. |
| Found: | C, | 52.06; | H, | 5.53; | N, | 17.77. |

## EXAMPLE II

Benzoic acid, [1aS-(1aα, 8β, 8aα, 8bα)]-[8-[[(aminocarbonyl)oxy]methyl]-1,1a,2,8,8a,8b-hexahydro-4-hydroxy-8a-methoxy-5-methyl-7 -oxoazirino[2 ,3 ,4]pyrrolo[1,2-a]indol-6(7H)-ylidene]hydrazide

Reacting mitomycin A with benzoic hydrazide (78.0 mg, 0.573 mmol) yielded 175 mg of a dark brown residue which on purification by chromatography (10% methanol-chloroform) gave 82 mg (63%) of a dark brown crystalline solid: mp >200 °C $R_f$ 0.30 (15% methanol-chloroform); IR (KBr) 1690, 1660, 1570, 1480, 1450, 1400, 1325, 1235, 1155 $cm^{-1}$; UV (MeOH) λ max 393 nm (ε 26800); $^1H$ NMR ($CD_3CN$) δ 2.10 (s, 3H), 2.79 (br s, 1H), 2.92 (br s, 1H), 3.20 (s, 3H), 3.52 (br dd, J=4.4, 10.9 Hz, 2H), 4.18 (br d, J=12.7 Hz, 2H), 4.84 (dd, J=4.4, 10.5 Hz, 1H), 7.54-7.64 (m, 3H), 7.93-7.95 (m, 2H), 17.03 (s, 1H); $^{13}C$ NMR (pyridine-$d_5$) 11.29, 32.61, 37.28, 43.89, 49.71, 50.73, 62.24, 106.93, 110.61, 121.71, 128.51, 129.24, 132.67, 133.91, 141.47, 143.09, 158.24, 160.01, 165.29, 174.97 ppm.

| | | | | | | |
|---|---|---|---|---|---|---|
| Anal. Calcd for $C_{22}H_{23}N_5O_6$. 0.9 $CHCl_3$: | C, | 49.03; | H, | 4.29; | N, | 12.48. |
| Found: | C, | 48.88; | H, | 4.85; | N, | 12.16. |

## EXAMPLE III

3-Pyridinecarboxylic acid, [1aS-(1aα, 8β, 8aα, 8bα)]-[8-[[(aminocarbonyl)oxy]methyl]-1,1a,2,8,8a,8b-hexahydro-4-hydroxy-8a-methoxy-5-methyl-7-oxoazirino[2 ,3 ;3,4,]pyrrolo[1,2-a]indol-6(7H)-ylidene]hydrazide

Utilizing nicotinic acid hydrazide (78.6 mg, 0.573 mmol) gave 175 mg of a dark brown colored residue which on purification by chromatography (15% methanol-chloroform) furnished 83 mg (64%) of the product as a dark brown crystalline solid: mp >200 °C; $R_f$ 0.17 (15% methanol-chloroform); IR (KBr) 1670, 1590, 1425, 1400, 1380, 1325, 1240, 1160, $cm^{-1}$; UV (MeOH) λ max 392 nm (ε 20000); $^1H$ NMR ($CD_3OD$) δ 2.10 (s, 3H), 2.88 (br s, 1H), 3.01 (br s, 1H), 3.25 (s, 3H), 3.62 (br dd, J=4.1, 10.9 Hz, 2H), 4.30 (dd, J=10.4, 10.9 Hz, 1H), 4.40 (dd, J=1.3, 12.5 Hz, 1H), 4.76 (dd, J=4.1, 10.4 Hz, 1H), 7.62 (dd, J=4.1, 7.8 Hz, 1H), 8.37 (dd, J=1.2, 7.8 Hz, 1H), 8.75 (d, J=4.1 Hz, 1H), 9.12, (br s, 1H); $^{13}C$ NMR ($CD_3OD$) 10.23, 33.45, 37.74, 43.80, 50.25, 50.82, 62.63, 107.38, 110.64, 121.77, 125.35, 130.26, 137.51, 143.33, 144.20, 149.97, 153.57, 150.60, 161.09, 165.66, 175.09 ppm.

| | | | | | | |
|---|---|---|---|---|---|---|
| Anal. Calcd for $C_{21}H_{22}N_6O_6$. 0.7 $CHCl_3$: | C, | 48.44; | H, | 4.25; | N, | 15.62. |
| Found: | C, | 48.59; | H, | 4.46; | N, | 15.41. |

## EXAMPLE IV

2-Thiophenecarboxylic acid, [1aS-(1aα, 8β, 8aα, 8bα)-[8-[[(aminocarbonyl)oxy]methyl]-1,1a,2,8,8a,8b-hexahydro-4-hydroxy-8a-methoxy-5-methyl-7 -oxoazirino[2 ,3 ;3,4]pyrrolo[1,2-a]indol-6(7H)-ylidene]-hydrazide

Using 2-thiophenecarboxylic acid hydrazide (81.5 mg, 0.573 mmol) gave 175 mg of a dark brown residue which was purified by column chromatography (12% methanol-chloroform). The unconsumed mitomycin A and 2-thiophenecarboxylic acid hydrazide which coeluted during the column chromatography were further reacted for another 5 days in methanol (10 mL) and the reaction mixture purified to give additional product. The desired compound was obtained as dark brown crystalline material in an overall

yield of 81% (106 mg): mp >200 °C; $R_f$ 0.27 (15% methanol-chloroform); IR (KBr) 1700, 1630, 1565, 1480, 1410, 1340, 1325, 1225, 1100 $cm^{-1}$; UV (MeOH) λ max 396 nm (ε 24400); $^1H$ NMR ($CD_3OD$) δ 2.16 (s, 3H), 2.88 (br s, 1H), 3.03 (br s, 1H), 3.25 (s, 3H), 3.63 (br dd, J=4.2, 11.0 Hz, 2H), 4.30 (br dd, J=10.5, 11.0 Hz, 2H), 4.77 (dd, J=4.2, 10.5 Hz, 1H), 7.23 (dd, J-3.7, 5.1 Hz, 1H), 7.89 (br d, J=5.1 Hz, 1H), 7.92 (d, J=3.7 Hz, 1H); $^{13}C$ NMR (pyridine-$d_5$) 11.69, 32.53, 37.26, 43.78, 49.62, 50.70, 62.16, 106.91, 110.60, 121.34, 128.03, 132.71, 133.64 136.38, 140.28, 143.11, 158.21, 159.88, 161.43, 174.82 ppm; mass spectrum m/e (relative intensity) (negative CI) 477 (M+18, 11), 475 (M+16, 100), 460 (M+1, 15), 459 (M, 34), 414 (18), 400 (8), 286 (22).

| | | | | | | |
|---|---|---|---|---|---|---|
| Anal. Calcd for $C_{20}H_{21}N_5O_6S$. 0.6 $CHCl_3$: | C, | 46.58; | H, | 4.09; | N, | 13.18. |
| Found: | C, | 46.57; | H, | 4.35; | N, | 13.22. |

## EXAMPLE V

Ethyl [1aS-(1aα, 8β, 8aα, 8bα)]-[8-[[(aminocarbonyl)oxy]methyl]-1,1a,2,8, 8a,8b-hexahydro-4-hydroxy-8a-methoxy-5-methyl-7-oxoazirino[2′,3′,3,4] pyrrolo[1,2-a]indol-6(7H)-ylidene]hydrazinecarboxylate

Employing 2 equivalents of ethyl carbazate (59.5 mg, 0.573 mmol) gave 160 mg of an orange brown solid which after purification by trituration yielded 76 mg (63%) of an orange crystalline solid: mp >200 °C; $R_f$ 0.52 (20% methanol-chloroform); IR (KBr) 1760, 1690, 1630, 1620, 1560, 1475, 1430, 1405, 1375, 1325, 1230, 1175 $cm^{-1}$; UV (MeOH) λ max 376 nm (ε 26300); $^1H$ NMR ($CD_3OD$) δ 1.33 (t, J=7.0 Hz, 3H), 2.08 (s, 3H), 2.87 (d, J=3.9 Hz, 1H), 3.01 (d, J=3.9 Hz, 1H), 3.23 (s, 3H), 3.59 (br dd, J=4.1, 10.7 Hz, 2H), 4.30 (br q, J=7.0 Hz, 4H), 4.70 (dd, J=4.1, 10.5 Hz, 1H); $^{13}C$ NMR (pyridine-$d_5$) 11.16, 14.52, 32.50, 37.37, 43.89, 49.56, 50.69, 62.05, 62.21, 106.87, 110.58, 121.56, 139.52, 142.24, 154.54, 158.21, 159.85, 174.95 ppm.

| | | | | | | |
|---|---|---|---|---|---|---|
| Anal. Calcd for $C_{18}H_{23}N_5O_7$: | C, | 51.30; | H, | 5.50; | N, | 16.62. |
| Found: | C, | 51.18; | H, | 5.44; | N, | 16.45. |

## EXAMPLE VI

1,1-Dimethylethyl [1aS-(1aα, 8β, 8aα, 8bα)]-[8-[[(aminocarbonyl)oxy]methyl1,1a, 2, 8, 8a, 8b-hexahydro-4-hydroxy-8a-methoxy-5-methyl-7--oxoazirino[2′, 3′;3, 4]pyrrolo[1,2-a]indol-6(7H)- ylidene]hydrazine-carboxylate

Using tert.-butylcarbazate (75.6 mg, 0.573 mmol) gave 175 mg of an orange brown residue which after purification by trituration and chromatography (10% methanol-chloroform) furnished 118 mg (72%) of an orange crystalline solid: mp >200 °C; $R_f$ 0.38 (15% methanol- chloroform); IR (KBr) 1720, 1610, 1475, 1330, 1240, 1140 $cm^{-1}$; UV (MeOH) λ max 377 nm (ε 28700); $^1H$ NMR ($CO_3OD$) δ 1.54 (s, 9H), 2.07 (s, 3H), 2.86 (dd, J=1.4, 4.6 Hz, 1H), 3.00 (d, J=4.6 Hz, 1H), 3.23 (s, 3H), 3.58 (br dd, J=4.1, 11.0 Hz, 2H), 4.28 (d, J=12.9 Hz, 1H), 4.30 (dd, J=10.4, 11.0 Hz, 1H), 4.69 (dd, J=4.1, 10.4 Hz, 1H); $^1H$ NMR (pyridine-$d_5$) δ 1.53 (s,9H), 2.30 (br d, J=5.5 Hz, 1H), 2.41 (s, 3H), 2.70 (br d, J=5.5 Hz, 1H), 3.23 (s, 3H), 3.69 (br d, J=12.7 Hz, 1H), 4.11 (dd, J=4.0, 10.8 Hz, 1H), 4.64 (d, J=12.7 Hz, 1H), 5.14 (dd, J=10.3, 10.8 Hz, 1H), 5.68 (dd, J=4.0, 10.3 Hz 1H); $^{13}C$ NMR (pyridine-$d_5$) 10.19, 28.39, 33.42, 37.99, 43.90, 50.09, 50.77, 62.70, 83.37, 107.20, 110.64, 122.79, 139.96, 142.24, 155.00, 159.61, 160.53, 175.00 ppm; mass spectrum, m/e (relative intensity) (CI) 450 (M+1, 35), 394 (28), 350 (43), 289 (33), 287 (27), 257 ( 33), 177 (62), 139 (97), 111 (100).

| | | | | | | |
|---|---|---|---|---|---|---|
| Anal. Calcd for $C_{20}H_{27}N_5O_7 . CHCl_3$: | C, | 44.34; | H, | 4.96; | N, | 12.31. |
| Found: | C, | 44.43; | H, | 4.96; | N, | 12.14. |

EXAMPLE VII

Phenylmethyl [1aS-(1aα, 8β, 8aα, 8bα)]-[8-[[(aminocarbonyl)-oxy]methyl]-1,1a, 2, 8, 8a, 8b-hexahydro-4-hydroxy-8a-methoxy-5-methyl-7-oxoazirino[2 , 3 ; 3, 4]pyrrolo[1,2-a]indol-6(7H)-ylidene] hydrazinecarboxylate

Using benzylcarbazate (95.0 mg, 0.573 mmol) furnished approximately 200 mg of an orange-brown residue which was purified by trituration and chromatography (8% methanol-chloroform). The unreacted mitomycin A and benzylcarbazate which coeluted from the chromatographic column were allowed to further react (6 days) in methanol (10 mL) to give an additional amount of the product. The desired product was obtained as orange crystals in 85% yield (117 mg): mp >200 °C; $R_f$ 0.30 (10% methanol-chloroform); IR (KBr) 1715, 1700, 1605, 1560, 1470, 1400, 1325, 1225, 1160 $cm^{-1}$; UV (MeOH) λ max 377 nm (ϵ 25300); $^1H$ NMR ($CD_3OD$) δ 2.06 (s, 3H), 2.85 (br s, 1H), 2.98 (br s, 1H), 3.22 (s, 3H), 3.58 (br dd, J=4.2, 11.1 Hz, 2H), 4.30 (br dd, J=10.6, 11.1 Hz, 2H), 4.69 (dd, J=4.2, 10.6 Hz, 1H), 5.28 (s, 2H) 7.32-7.43 (m, 5H); $^{13}C$ NMR (pyridine-$d_5$) 11.08, 32.49, 37.32, 43.81, 49.54, 50.65, 62.18, 67.58, 106.86, 110.41, 121.41, 128.52, 128.89, 136.80, 137.69, 139.77, 142.34, 154.53, 158.17, 159.85, 174.87 ppm; mass spectrum, m/e (relative intensity) (negative CI) 501 (M+18, 7), 499 (M+16, 100), 483 (M, 38), 467 (7), 439 (7), 438 (22).

| | | | | | | |
|---|---|---|---|---|---|---|
| Anal. Calcd for $C_{23}H_{25}N_5O_7 . 0.1CHCl_3$: | C, | 56.00; | H, | 5.10; | N, | 14.13. |
| Found: | C, | 55.88; | H, | 5.17; | N, | 13.76. |

EXAMPLE VIII

9′-Fluorenylmethyl[1aS-(1aα, 8β, 8aα, 8bα)]-[8-[[(aminocarbonyl) oxy]methyl]-1, 1a, 2, 8, 8a, 8b-hexahydro-4-hydroxy-8a-methoxy-5-methyl-7-oxoazirino[2 , 3 ;3,4,]pyrrolo[1,2-a] indol-6(7H)-ylidene]hydrazine carboxylate

Making use of 9-fluorenylmethyl carbazate (145.5 mg, 0.573 mmol) gave 240 mg of an orange brown residue which after purification by chromatography (10% methanol-chloroform) furnished 67 mg (41%) of an orange solid: mp>200 °C; $R_f$ 0.14 (10% methanol-chloroform); IR (KBr) 1700, 1610, 1560, 1470, 1445, 1400, 1340, 1320, 1270, 1160 $cm^{-1}$; UV (MeOH) λ max 263 (ϵ, 18400), 298 sh (ϵ, 8000), 377 (ϵ, 22900); $^1H$ NMR ($CD_3OD$) δ 2.09 (s, 3H), 2.87 (br s, 1H), 3.00 (br s, 1H), 3.25 (s, 3H), 3.61 dd, J=3.9, 10.9 Hz, 2H), 4.29-4.34 (m, 2H), 4.54-4.61 (m, 3H), 4.72 (dd, J= 3.9, 10.3 Hz, 1H), 7.30-7.43 (m 4H), 7.69 (d, J=7.3 Hz, 2H), 7.82 (d, J=7.3 Hz, 2H); $^{13}C$ NMR (pyridine-$d_5$) 10.94, 32.14, 37.15, 43.68, 47.20, 49.46, 50.51, 62.05, 68.08, 106.70, 110.34, 120.34, 121.35, 125.66, 127.47, 128.10, 139.60, 141.52, 144.01, 144.10, 154.43, 158.19, 159.82, 174.66 ppm; mass spectrum.

| | | | | | | |
|---|---|---|---|---|---|---|
| Anal. Calcd for $C_{30}H_{29}N_5O_7 . H_2O$: | C, | 61.11; | H, | 5.30; | N, | 11.88. |
| Found: | C, | 61.43; | H, | 4.98; | N, | 11.80. |

In like manner, other substituted hydrazines, X-NH-NH-, can be attached to the 7-position of mitomycin derivatives, by reaction of X-NH-$NH_2$ with mitomycin A derivatives.

In defining the R or $R_1$ substituents, the following terms are defined by way of example, not limitation:

The alkyl groups exemplary of the substituents are lower alkyl containing from 1 to 6 carbon atoms and

may be straight chain or branched. These groups include methyl, ethyl, propyl, isopropyl, butyl, isobutyl, tertiary butyl, amyl, hexyl, and the like.

The aryl lower alkyl groups include, for example, benzyl, phenethyl, phenpropyl, phenisopropyl, phenbutyl, and the like, diphenylmethyl, 1,1-diphenylethyl 1,2-diphenylethyl, and the like.

Exemplary of the unsaturated alkyl substituents, i.e., lower alkenyl and lower alkynyl, are vinyl, acetylenic, allyl, propenyl, butenyl, pentenyl, hexenyl, propynl, butynl, pentynl, hexynl, pentadienyl, and the like.

The heterocylic substituents contemplated by the present invention include heteroaromatics and saturated and partially unsaturated heterocylic compounds such as furyl, thienyl, pyranyl, pyrrolyl, imidazoyl, pyrazolyl, pyridyl, prazinyl, pyrimidyl, pyridazinyl, indolyl, thiazolyl, oxazolyl, isothiazolyl, isoxazolyl, piperidyl, pyrrolinyl, piperazinyl, quinolyl, triazolyl, tetrazolyl, and the like.

The polynuclear aromatic substituents contemplated herein include, for example, naphthyl, anthracenyl, phenanthrenyl, azulenyl, and the like.

The heteroatom containing substituents include, for example, methoxy, ethoxy, phenoxy, thiomethoxy, thioethoxy, thiophenoxy, methylamino, ethylamino, anilino, dimethlamino, trimethylamino, fluoro, chloro, bromo, iodo, and the like.

The aryl groups such as phenyl, ferrocenyl, and the like, the alkyl groups, the aryl lower alkyl groups, the lower alkenyl group, the aryl lower alkenyl group, the lower alkynyl groups, the aryl lower alkynyl group, and heterocylic, heterocylic lower alkyl, polynuclear aromatic, and polynuclear aromatic lower alkyl may carry one or more substituents such as halo, including bromo, fluoro, chloro, iodo, and the like, nitro, acyl, carboxyl, carboalkoxy, carboxamide, cyano, sulfonyl, sulfoxide, heterocyclic, quanidine, quarternary ammonium, and the like; or hydroxy, alkoxy including methoxy, ethoxy, and the like, alkyl, amino, substituted amino, phenoxy, substituted phenoxy, thiol, sulfide, disulfide, and the like.

## Activity Against P-388 Murine Leukemia

The Table contains the results of laboratory tests with $CDF_1$ female mice implanted intraperitoneally with a tumor inoculum of $10^6$ ascites cells of P-388 murine leukemia and treated with various doses of either a test compound of this invention, or with mitomycin C. The compounds were administered by intraperitoneal injection. Groups of six mice were used for each dosage amount and they were treated with a single dose of the compound on the day of inoculation. A group of ten saline treated control mice was included in each series of experiments. The mitomycin C treated groups were included as a positive control. A 30-day protocol was employed with the mean survival time in days being determined for each group of mice and the number of survivors at the end of the 30-day period being noted.

The results were determined in terms of % T/C which is the ration of the mean survival time of the treated group to the mean survival time of the saline treated control group times 100. The saline treated control animals usually died within nine days. The "maximum effect" in the following Table is expressed as % T/C and the dose giving that effect is given in parentheses. The values in brackets are the values obtained with mitomycin C as the positive control in the same experiment. Thus, a measure of the relative activity of the present substances to mitomycin C can be estimated. A minimum effect in terms of % T/C was considered to be 125. The minimum effective dose (MED) reported in the following Table is that dose given a % T/C of approximately 125 or greater.

The data for the in vivo tests with mitomycin C derivatives I-VIII against P-388 leukemia are listed in the Table. For the purpose of discussion, the compounds evaluated can be classified as either acid hydrazide (i.e., I-IV) or as carbazate (i.e., V-VIII) mitomycin C analogs. All the compounds were biologically active. Within the carbazate series, adducts V, VII and VIII displayed the highest efficacy (optimum T/C%) when compared to the concurrently run mitomycin C controls. Of the remaining acyl hydrazide mitomycin C derivatives, only the acethydrazide adduct I exhibited pronounced activity (T/C% = 190, 51.2 mg/kg per dose), while the three aroyl hydrazide adducts II, III and IV were moderately active.

TABLE

| IN VIVO TEST RESULTS FOR MITOMYCIN C DERIVATIVES I - VIII[a] | | |
|---|---|---|
| No. | P-388 screen[b] | |
| | Maximum[c] %T/C | MED[d] |
| I | 190(51.2)[205(4.8)] | 1.6[1.6] |
| II | 150(25.6)[205(4.8)] | 0.8[0.8] |
| III | 150(25.6)[205(4.8)] | 12.8[1.6] |
| IV | 140(6.4)[205(4.8)] | 0.8[0.8] |
| V | 180(25.6)[205(4.8)] | 0.8[0.8] |
| VI | 160(25.6)[205(4.8)] | 0.8[0.8] |
| VII | 185(6.4)[205(4.8)] | 1.6[1.6] |
| VIII | 195(25.6)[215(4.8)] | 0.8[1.6] |

[a] Tests conducted at Bristol-Myers Laboratories.
[b] P-388 lymphocytic leukemia screen: the tumor was ip as was the treatment.
[c] The values reported are the median survival time for the test substrate at the optimal dose listed in parentheses. The corresponding values for the concurrently run mitomycin C sample appear in brac ets. %T/C = (median survival time of drug treated/median survival time of control) X 100.
[d] The values reported are minimum effective dose (%T/C≥125) in mg/kg per dose observed for the substrate tested, followed by corresponding values for mitomycin C in brackets. MED = minimum effective dose.

Substituted hydrazine-7-mitomycin derivatives, as hereinbefore defined, and physiologically acceptable salts thereof may be used as antibacterial and anti-tumor agents owing to their antibacterial and anti-tumor activities.

Thus, according to the present invention, there are provided pharmaceutical compositions containing as active ingredient at least one substituted hydrazine-7-mitomycin derivative or a physiologically acceptable salt thereof in association with one or more pharmaceutical carries and/or excipients. Generally, the preferred substituted hydrazine-7-mitomycin derivatives and physiologically acceptable salts thereof for use as medicaments are those with high water solubility.

For use as, for example, anti-tumor agents, substituted hydrazine-7-mitomycin derivatives may be dissolved, for example, in physiological saline solution, or a glucose, lactose or mannitol injection solution. Administration may, for example, be effected by intravenous injection at a dose of 0.02-1mg/kg of body weight. Compounds may be freeze-dried and a dry powder injectable formulation may be prepared with addition of sodium chloride. The anti-tumor agent may further contain therein well-known, pharmacologically acceptable diluent(s), adjuvant(s) and/or carrier(s) such as salts which fulfill pharmaceutical utility. The dose required of a pharmaceutical composition according to the invention may be varied depending upon, for example, the age and symptoms of each patient. The administration schedule may be varied depending upon the dose. Thus, administration may be effected, for example, once a week or once every three weeks.

## Claims

1. A mitomycin derivatives having a substituted hydrazine of the formula X-NH-NH- at position 7 of the mitomycin ring, wherein X is selected from the group consisting of:

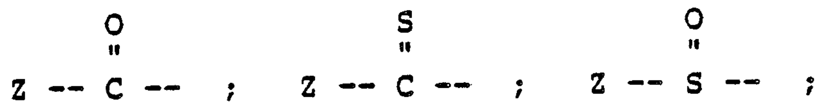

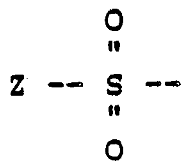 and 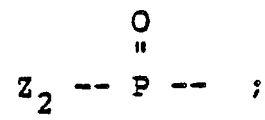

wherein Z is R or RY;
wherein R is hydrogen, lower alkyl, lower alkenyl, lower alkynyl, aryl lower alkyl, aryl lower alkenyl, aryl lower alkynyl, aryl, heterocylic, heterocylic lower alkyl, polynuclear aromatic or polynuclear aromatic lower alkyl, each unsubstituted or substituted with one or more halogen or a heteroatom which is oxygen, nitrogen, sulfur or phosphorous substituted with hydrogen, lower alkyl or aryl; carbohydrate; amino acid; nucleic acid; oligo peptide or protein; and
Y is NH, O, or S.

2. A mitomycin derivative having the formula

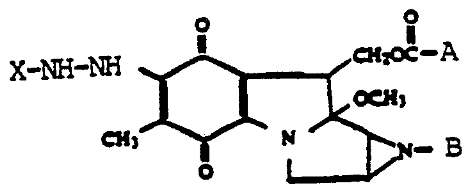

or its corresponding tautomeric form,
wherein:
X is selected from the group consisting of:

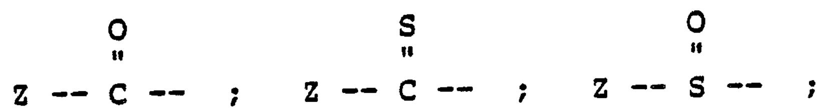

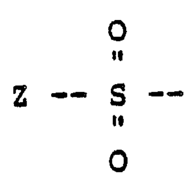 and 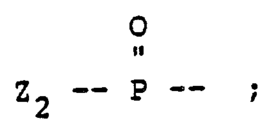

wherein Z is R or RY;
wherein R is hydrogen, lower alkyl, lower alkenyl, lower alkynyl, aryl lower alkyl, aryl lower alkenyl, aryl lower alkynyl, aryl, heterocyclic, heterocyclic lower alkyl, polynuclear aromatic or polynuclear aromatic lower alkyl, each unsubstituted or substituted with one or more halogen or a heteroatom which is oxygen, nitrogen, sulfur or phosphorous substituted with hydrogen, lower alkyl or aryl; carbohydrate; amino acid; nucleic acid; oligo peptide or protein; and
Y is NH, O, or S;
A is $R_1$ or $YR_1$, wherein $R_1$ includes:
hydrogen, lower alkyl, lower alkenyl, lower alkynyl, aryl, each unsubstituted or substituted with one or more of halogen or a heteroatom which is oxygen, nitrogen, sulfur or phosphorous substituted with hydrogen lower alkyl or aryl;

and Y is NH, O, or S; and

$$B \text{ is } R_1\ ;\ -C(=O)-R_1\ ;\ -C(=S)-R_1\ ;$$

$$-C(=O)-YR_1\ ;\ -C(=S)-YR_1\ ;\ -P(=O)-(YR_1)_2\ ;$$

$$-P(=O)-(R_1)_2\ ;\ -S(=O)_2-R_1\ ;\ -S(=O)_2-YR_1\ ;\ \text{or}\ -S(=O)-R_1\ .$$

3. The mitomycin derivative of claim 1 wherein X is $Z-C(=O)-$.

4. The mitomycin derivatives of claim 1 wherein X is acetyl; benzoyl; 3-pyridine carboxy; 2-thiophenyl carboxy; carbethoxy; carbo*tert*-butoxy; carbobenzoxy; or carbo-9-fluoromethloxy.

5. The mitomycin derivatives of claim 2 wherein A is amino and B is hydrogen.

6. The mitomycin derivative of claim 2 wherein X is acetyl; benzoyl; 3-pyridine carboxy; 2-thiophenyl carboxy; carbethoxy; carbo*tert*-butoxy; carbobenzoxy; or carbo-9-fluoromethloxy; A is amino and B is hydrogen.

7. A composition comprising a substituted hydrazine-7-mitomycin derivative of claim 1 or a physiologically acceptable salt thereof and a pharmaceutical carrier.

8. A method of treating neoplastic disease in a host comprising administering to the host an antineoplastic effective amount of a substituted hydrazine-7-mitomycin derivative of claim 1 or a physiologically acceptable salt thereof.

European Patent Office

# PARTIAL EUROPEAN SEARCH REPORT

which under Rule 45 of the European Patent Convention shall be considered, for the purposes of subsequent proceedings, as the European search report

Application number: EP 89102266.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A2 - 0 246 094 (KYOWA)<br>* Claims; examples 17-33, tables 3,4 *<br>-- | 1-7 | C 07 D 487/14<br>A 61 K 31/40<br>(C 07 D 487/14<br>C 07 D 209/00<br>C 07 D 209/00<br>C 07 D 203/00) |
| A | EP - A2 - 0 191 375 (KYOWA)<br>* Abstract; claims; examples *<br>-- | 1-7 | |
| A | GB - A - 2 121 796 (BRISTOL-MYERS)<br>* Abstract; claims; examples *<br>-- | 1-7 | |
| D,A | JOURNAL OF MEDICINAL CHEMISTRY, vol. 26, no. 7, 1983<br>B.S.IYENGAR et al. "Mitomycin C analogues with secondary amines at position 7"<br>pages 1453-7<br>* Totality; especially table 1 *<br>---- | 1-7 | TECHNICAL FIELDS SEARCHED (Int. Cl.4)<br>C 07 D 487/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7

Claims searched incompletely: –

Claims not searched: 8

Reason for the limitation of the search:

(Method for the treatment of the human or animal body by therapy; Art. 52(4) EPC)

| Place of search | Date of completion of the search | Examiner |
|---|---|---|
| VIENNA | 12-04-1989 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1. 03.82